(19) Europäisches Patentamt
European Patent Office
Office européen des brevets

(11) **EP 3 967 222 B1**

(12) **EUROPEAN PATENT SPECIFICATION**

(45) Date of publication and mention
of the grant of the patent:
**11.09.2024 Bulletin 2024/37**

(21) Application number: **20804767.0**

(22) Date of filing: **14.04.2020**

(51) International Patent Classification (IPC):
*A61B 5/08* *(2006.01)* *A61B 5/087* *(2006.01)*
*G01F 1/34* *(2006.01)*

(52) Cooperative Patent Classification (CPC):
**A61B 5/0803; A61B 5/087; G01F 1/363; G01F 1/42**

(86) International application number:
**PCT/KR2020/005016**

(87) International publication number:
**WO 2020/231019 (19.11.2020 Gazette 2020/47)**

(54) **RESPIRATION MEASUREMENT DEVICE**

ATMUNGSMESSVORRICHTUNG

DISPOSITIF DE MESURE DE RESPIRATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **10.05.2019 KR 20190054708**

(43) Date of publication of application:
**16.03.2022 Bulletin 2022/11**

(73) Proprietor: **Breathings Co., Ltd.
Gangwon-do 26354 (KR)**

(72) Inventors:
• **LEE, In Pyo
Seoul 06249 (KR)**
• **YOON, Ki Sang
Yongin-si Gyeonggi-do 17002 (KR)**
• **SONG, Chang Ho
Seongnam-si Gyeonggi-do 13584 (KR)**

(74) Representative: **dompatent von Kreisler Selting
Werner -
Partnerschaft von Patent- und Rechtsanwälten
mbB
Deichmannhaus am Dom
Bahnhofsvorplatz 1
50667 Köln (DE)**

(56) References cited:
EP-A1- 0 552 916        WO-A2-2018/073343
JP-A- 2017 525 512      JP-A- 2018 528 803
KR-A- 20040 100 412     KR-A- 20160 026 464
KR-A- 20180 105 404     KR-B1- 102 039 984
US-A- 5 137 026         US-A- 5 676 132

**Description**

[Technical Field]

The present invention relates to a respiration measurement device, and more particularly to a respiration measurement device which has a reduced size so that the respiration volume of an examinee can be measured anytime and anywhere as well as at home or in a hospital, and allows a sensor configured to measure respiratory air flow and an air flow path configured to allow the respiratory air flow to be moved therethrough to be separately provided and then to be detachably coupled to each other, so that foreign substances produced in the sensor and the air flow path can be easily washed and removed.

[Background Art]

A prior art respiration measurement device is disclosed in US 5 137 026.

In general, measurement devices used in pulmonary function tests are divided into two methods. In the first method, changes in a lung volume due to inflation and deflation of the lung, i.e., changes in the lung volume, are measured quantitatively, and more particularly, changes in the lung volume are directly measured while an examinee respires in a predetermined mode. In the second method, the respiration volume of an examinee is measured by sensing and measuring the flow of air flowing towards the inside and the outside of the lung while the examinee respires.

Conventionally, the first method in which changes in the lung volume are directly measured was mainly used in pulmonary function tests but a test device used in this method has a complicated structure and is difficult to move and thus causes inconvenience in use, and therefore, the second method in which respiratory air flow generated by respiration of an examinee is measured and a respiration measurement device using this method are mainly used now.

However, the above respiration measurement device configured to measure the respiratory air flow generally has a complicated inner structure and a very large size, and is thus difficult to carry around and may be used only at home or in a hospital.

Recently, air pollution caused by sandy dust, heavy metals and fine particles is rapidly increasing, infants, the old and the weak are very vulnerable to respiratory diseases in these circumstances, and thus, it is very important to continuously check the healthy state of the respiratory organs through measurement of the respiration volume.

However, there is a problem in that it is almost impossible to rapidly, conveniently and accurately measure the respiration volume of an examinee in an environment deviating from a hospital or home.

Further, in the conventional respiration measurement device, a sensor configured to measure respiratory air flow and an air flow path configured to allow the respiratory air flow to be moved therethrough are formed integrally with each other, and thus, it is difficult to wash and remove foreign substances produced in the sensor and the air flow path.

[Disclosure]

[Technical Problem]

Therefore, the present invention has been made in view of the above problems, and it is an object of the present invention to provide a respiration measurement device which has a reduced size so that the respiration volume of an examinee can be measured anytime and anywhere as well as at home or in a hospital, and allows a sensor configured to measure respiratory air flow and an air flow path configured to allow the respiratory air flow to be moved therethrough to be separately provided and then to be detachably coupled to each other, so that foreign substances produced in the sensor and the air flow path can be easily washed and removed. It is another object of the present invention to provide a respiration measurement device which may increase sensitivity while having a simplified structure.

[Technical Solution]

In accordance with the present invention, the above and other objects can be accomplished by the provision of a respiration measurement device including a first body configured to have an air flow path formed therein to allow air generated by respiration of a user to be introduced thereinto and moved therethrough, a part of the air flow path being opened to form an opening, a second body including a respiration measurement unit configured to measure a pressure of the air moved through the air flow path, formed to be detachably coupled to the first body and installed to close the opening formed through the first body, and a mouthpiece detachably coupled to an inlet side of the air flow path.

Here, the respiration measurement unit includes a respiration measurement sensor module formed to correspond to the opening of the air flow path and installed to close the opening of the air flow path by coupling between the first body and the second body, and a first differential pressure generation block configured to extend from the respiration measurement sensor module towards an inner center of the air flow path.

Further, the respiration measurement device further includes a second differential pressure generation block disposed on the inner surface of the air flow path, configured to extend from an inner surface of the air flow path towards the inner center of the air flow path, and disposed opposite the first differential pressure generation block so as to correspond to the first differential pressure generation block.

The respiration measurement sensor module may include a sensing flow path configured to pass through an inside of the respiration measurement sensor module and provided with an inlet and an outlet formed at both sides of the first differential pressure generation block, and a sensing unit configured to sense a pressure difference between the inlet and the outlet of the sensing flow path.

The respiration measurement device may further include a mouthpiece coupling member installed between the mouthpiece and an inlet side of the air flow path, a metal body may be formed at one side of the mouthpiece coupling member, and a magnetic body may be formed at the mouthpiece so as to be detachably coupled to the metal body.

Further, the respiration measurement device may further include a filter installed in the mouthpiece coupling member so as to filter out contaminants from the inlet side of the air flow path.

The respiration measurement device may further include a respiratory pressure adjustment unit disposed between the inlet side of the air flow path and the mouthpiece coupling member so as to adjust the pressure of respiratory air flow introduced into the air flow path by rotation.

The respiratory pressure adjustment unit may include a rotating member provided with a rotating shaft formed at a center thereof and installed to be rotatable by the rotating shaft, and a plurality of pressure adjustment holes installed to pass through the rotating member and disposed radially about the rotating shaft.

The pressure adjustment holes may be configured such that cross-sectional areas thereof are different.

[Advantageous Effects]

A respiration measurement device according to the present invention has a reduced size so that the respiration volume of an examinee can be measured anytime and anywhere as well as at home or in a hospital, and allows a sensor configured to measure respiratory air flow and an air flow path configured to allow the respiratory air flow to be moved therethrough to be separately provided and then to be detachably coupled to each other, so that foreign substances produced in the sensor and the air flow path can be easily washed and removed. Further, one or more differential pressure generation blocks are formed in the air flow path, and thereby, the respiration measurement device may increase sensitivity while having a simplified structure. A respiratory pressure adjustment unit adjusts the pressure of the respiratory air flow introduced into the air flow path, and may thus provide a respiratory pressure suitable for the user's health condition during user's respiratory movement, thereby being capable of improving the respiratory movement effects of individuals.

[Description of Drawings]

FIGs. 1 and 2 are perspective views of a respiration measurement device according to the present invention.
FIG. 3 is a plan view illustrating a first body of the respiration measurement device according to the present invention.
FIG. 4 is a schematic view of the respiration measurement device according to the present invention.
FIG. 5 is a cross-sectional view of the respiration measurement device according to the present invention.
FIG. 6 is a view illustrating a respiration measurement unit of the respiration measurement device according to the present invention.
FIG. 7 is a view illustrating a respiratory pressure adjustment unit of the respiration measurement device according to the present invention.
FIG. 8 is a view illustrating a mouthpiece of the respiration measurement device according to the present invention.
FIG. 9 is a view illustrating a filter of the respiration measurement device according to the present invention.
FIG. 10 is a view illustrating a principle of measuring a respiration volume using the respiration measurement device according to the present invention.

[Mode for Invention]

Specific structural or functional descriptions in embodiments of the present invention set forth in the description which follows will be exemplarily given to describe the embodiments of the present invention, and the present invention may be embodied in many alternative forms and should not be construed as being limited to the embodiments set forth herein.

The embodiments of the present invention may be variously modified and changed, and thus specific embodiments of the present invention will be illustrated in the drawings and described in detail in the following description of the embodiments of the present invention. However, it will be understood that the embodiments of the present invention are provided only to completely disclose the invention and cover modifications, equivalents or alternatives which come within the scope and technical range of the invention.

In the following description of the embodiments, terms, such as "first" and "second", are used only to describe various elements, and these elements should not be construed as being limited by these terms. These terms are used only to distinguish one element from other elements, and for example, a first element described hereinafter may be termed a second element, and similarly, a second element described hereinafter may be termed a first element, without departing from the scope of the invention.

When an element is referred to as being "connected to" or "coupled to" another element, it may be directly connected or coupled to the other element, or intervening elements may be present. In contrast, when an element is referred to as being "directly connected to" or "directly coupled to" another element, there may be no intervening elements present. Other words used to describe relationships between elements should be interpreted in a like fashion, e.g., "between" versus "directly between," "adjacent" versus "directly adjacent," etc.

The terminology used herein is for the purpose of describing particular embodiments only and is not intended to be limiting. As used herein, singular forms may be intended to include plural forms as well, unless the context clearly indicates otherwise. The terms "comprising", "having", etc. are inclusive and therefore specify the presence of stated features, integers, steps, operations, elements, components, and/or combinations thereof, but do not preclude the presence or addition of one or more other features, integers, steps, operations, elements, components, and/or combinations thereof.

Unless defined otherwise, all terms including technical and scientific terms used in the following description have the same meanings as those of terms generally understood by those skilled in the art. Terms defined in generally used dictionaries will be interpreted as having meanings coinciding with contextual meanings in the related technology, and are not to be interpreted as having ideal or excessively formal meanings unless defined clearly in the description.

Hereinafter, a respiration measurement device according to a preferred embodiment of the present invention will be described in detail with reference to the accompanying drawings.

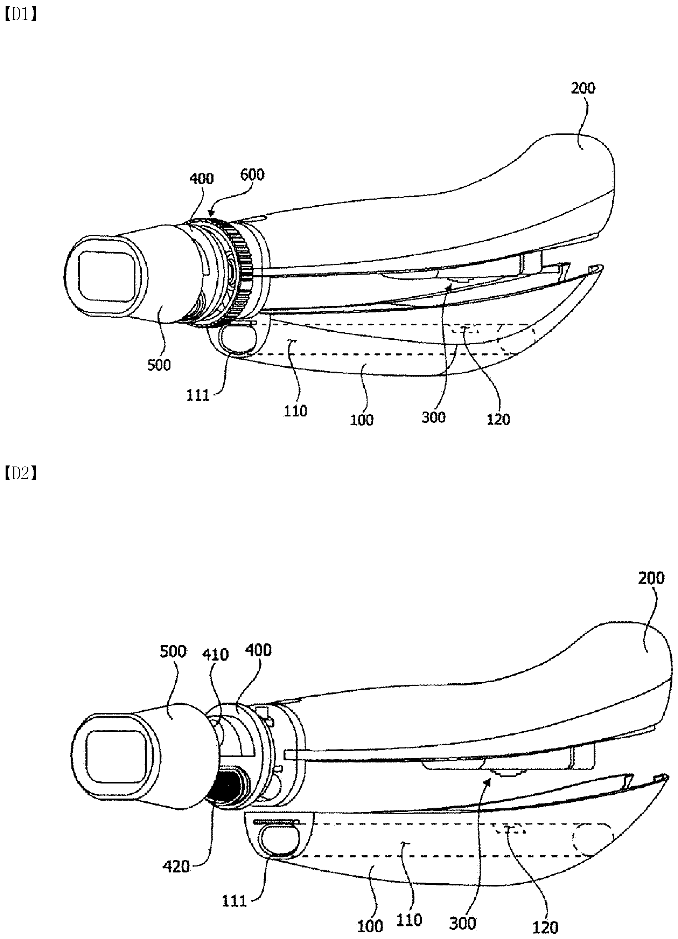

The respiration measurement device according to the present invention includes, as shown in FIGs. 1 to 4, a first body 100 configured to have an air flow path 110 formed therein to allow air generated by respiration of a user to be introduced thereinto and moved therethrough, a part of the air flow path 110 being opened to form an opening 120, a second body 200 formed to be detachably coupled to the first body 100 and installed to close the opening 120 formed through the first body 100, a respiration measurement unit 300 configured to measure the pressure of the air moved through the air flow path 110, a mouthpiece coupling member 400 installed at an inlet side 111 of the air flow path 110, and a mouthpiece 500 detachably coupled to the mouthpiece coupling member 400.

First, the first body 100 is a hollow member in which the air flow path 110 is formed, and one side (a left side in FIG. 1, i.e., an air inlet side) and the other side (a right side in FIG. 1, i.e., an air outlet side) of the first body 10 are open so that the air flow path 110 communicates with the outside. Further, the opening 120 is formed by removing a part of the upper surface of the first body 100, and thereby, the part of the upper surface of the air flow path 110 is open.

Further, the second body 200 is formed to have a length corresponding to the length of the first body 100, and the lower part of the second body 200 is detachably coupled to the first body 100.

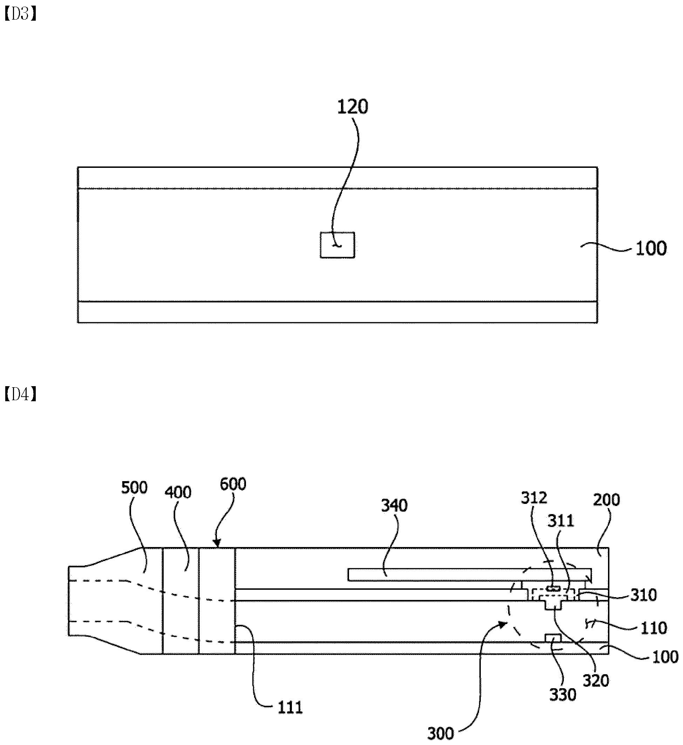

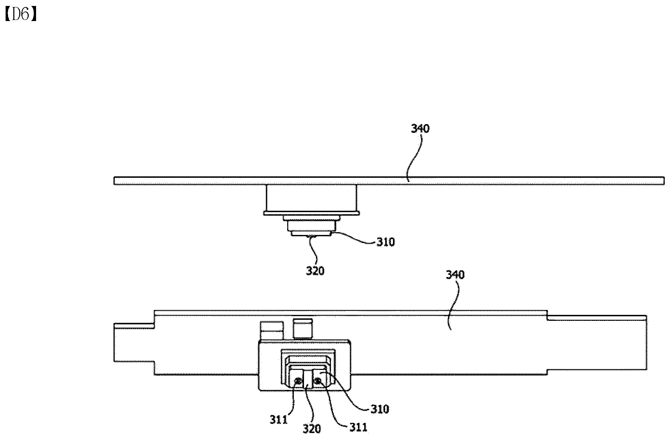

The respiration measurement unit 300 includes, as shown in FIGs. 4 to 6, a respiration measurement sensor module 310 formed to correspond to the opening of the air flow path and installed to close the opening of the air flow path by coupling between the first body and the second body, a first differential pressure generation block 320 configured to extend from the respiration measurement sensor module 310 towards the inner center of the air flow path, and a second differential pressure generation block 330 disposed on the inner surface of the air flow path, configured to extend from the inner surface of the air flow path towards the inner center of the air flow path, and disposed opposite the first differential pressure generation block so as to correspond to the first differential pressure generation block.

The respiration measurement sensor module 310 is a member formed to the correspond to the opening 120 of the first body 100, and the respiration measurement sensor module 310 closes the opening 120 when the first body 100 and the second body 200 are coupled to each other. That is, the respiration measurement sensor module 310 covers the opening 120 by coupling between the first body 100 and the second body 200, thereby sealing the air flow path 110. As described above, since the first body 100 and the second body 200 may be formed to be completely separated from each other and a part of the air flow path 110 may be open by separation between the first body 100 and the second body 200, foreign substances may be easily removed from the inside of the air flow path 110 by washing the first body. Here, the respiration measurement sensor module 310 is disposed such that the lower surface of the respiration measurement sensor module 310 is located to be coplanar with the upper surface of the air flow path 110.

The first differential pressure generation block 320 is a member having a designated thickness and height, is disposed at the center of the respiration measurement sensor module 310, and protrudes from the surface of the respiration measurement sensor module 310 towards the inner center of the air flow path 110.

The respiration measurement sensor module 310 may include a sensing flow path configured to pass through the inside of the respiration measurement sensor module and provided with an inlet and an outlet formed at both sides of the first differential pressure generation block, and a sensing unit configured to sense a pressure difference. More concretely, the sensing flow path 311 configured to measure the pressure of air passing through the air flow path is formed in the respiration measurement sensor module 310, and the inlet and the outlet of the sensing flow path 311 are formed at both sides of the first differential pressure generation block 320 in the direction of air flow. Further, the sensing unit 312 is disposed in the sensing flow path 311. The sensing unit 312 is a differential pressure sensor, and may sense a pressure difference between the inlet and the outlet of the sensing flow path 311 and calculate a respiration volume based on the sensed pressure difference.

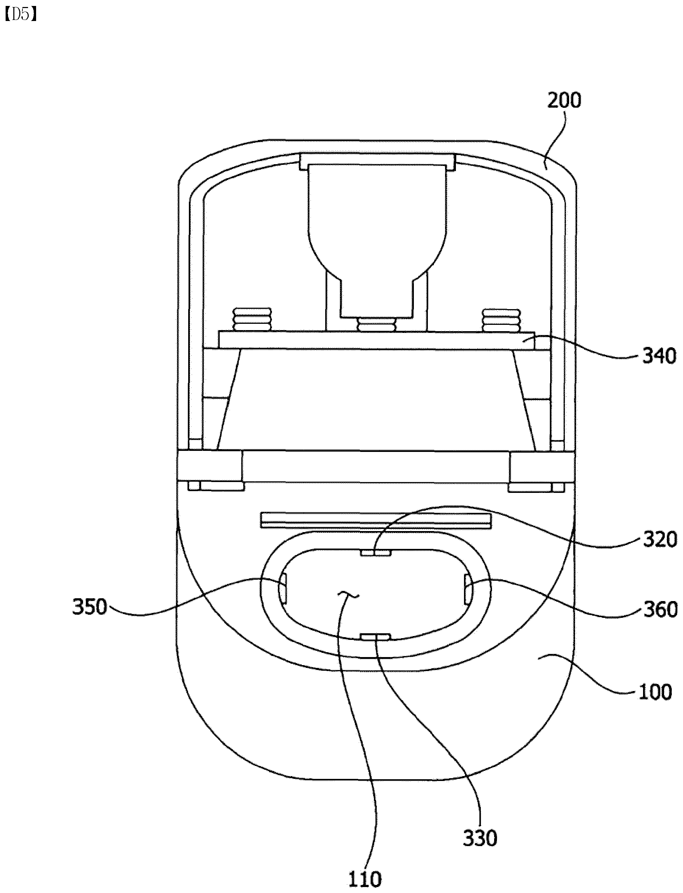

The respiration measurement sensor module 310 is installed on a circuit board 340, and the circuit board 340 is mounted in the second body 200. The respiration measurement sensor module 310 is installed to be exposed from the second body so as to close the opening 120 of the first body when the second body and the first body are coupled to each other (with reference to FIGs. 4 and 5).

Further, the second differential pressure generation block 330 is provided as a member having a designated thickness and height and formed to correspond the first differential pressure generation block 320, and is disposed on the inner surface of the air flow path 110. Here, the second differential pressure generation block 330 is disposed so as to face the first differential pressure generation block 320.

Further, the respiration measurement unit 300 according to the present invention may further include a third differential pressure generation block 350 and a fourth differential pressure generation block 360. The third differential pressure generation block 350 and the fourth differential pressure generation block 360 may be formed at both sides of the air flow path 110, as shown in FIG. 5.

The first to fourth differential pressure generation blocks are members having a shape having a designated thickness and height and, although the first to fourth differential pressure generation blocks have a rectangular shape in the present invention, the first to fourth differential pressure generation blocks may have various other shapes as long as they have a designated thickness and height and generate a differential pressure, as described above.

The respiration measurement unit 300 having the above-described configuration measures respiration of a user.

Hereinafter, the principle of the respiration volume of a user using the respiration measurement unit 300 according to the present invention will be described in detail.

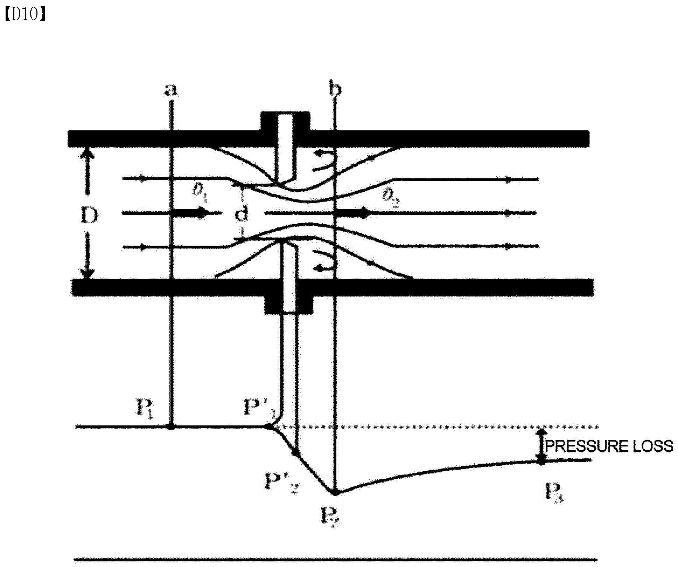

FIG. 10 is a view illustrating pressure changes in an orifice pipe installed in a general flow path.

Referring to FIG. 10, Bernoulli's equation and the continuity equation are established between the cross-section a of an upstream region of the orifice pipe and the cross-section b of a narrowed region of the orifice pipe.

$$P_1 + \frac{\rho_1 V_1^2}{2} = P_2 + \frac{\rho_2 V_2^2}{2}$$

$$\rho_1 V_1 A_1 = \rho_2 V_2 A_2$$

(V: mean flow velocity, P: pressure, $\rho$: density of fluid)

The relationship between a volume flow rate Q of the fluid passing through the orifice pipe and a differential pressure $(P_1\text{-}P_2)$ is as follows.

$$Q = V_2 A_2 = A_2 \frac{1}{(1-(A_2/A_1)^{1/2})} \left[\frac{1}{\rho_1}(P_1 - P_2)\right]^{1/2}$$

Therefore, when the values of $P_1$ and $P_2$ are given, the volume flow rate Q may be obtained.

In the present invention, the first differential pressure generation block 320 and the second differential pressure generation block 330 are substituted for the orifice pipe having the above-described measurement principle, and the values of $P_1$ and $P_2$ may be obtained by sensed results of the sensing unit 312 located in the sensing flow path 311.

When the obtained values of $P_1$ and $P_2$ are substituted into the above equation, the respiration volume of the user may be measured.

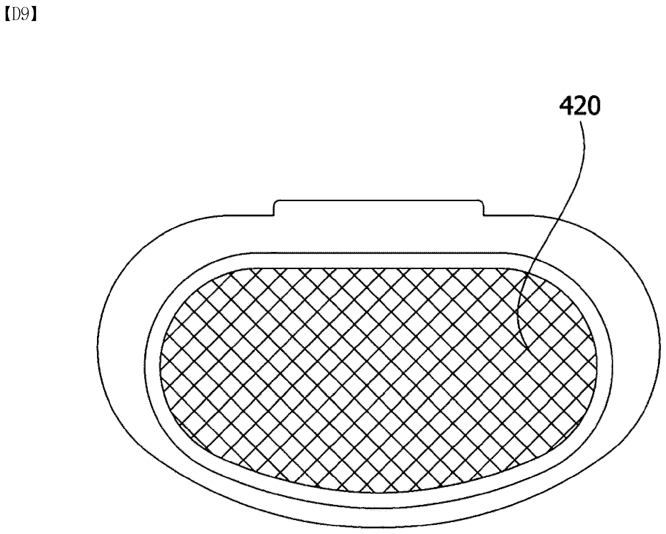

The mouthpiece coupling member 400 may be provided between the first body and the mouthpiece. The mouthpiece coupling member 400 is provided as a plate having a designated thickness, installed at the inlet side 111 of the air flow path 110, and coupled to the mouthpiece. Here, a metal body 410 is formed at one side of the mouthpiece coupling member 400 so as to be coupled to the mouthpiece 500, and a filter 420 is formed so as to be interlocked with the inlet side 111 of the air flow path 110 (with reference to FIG. 9). The filter 420 is provided as a general metal net or prefilter, and is installed so as to filter out foreign substances from the inlet side 111 of the air flow path 110. The mouthpiece coupling member 400 may be provided separately from the first body, or may be formed integrally with the first body, as needed.

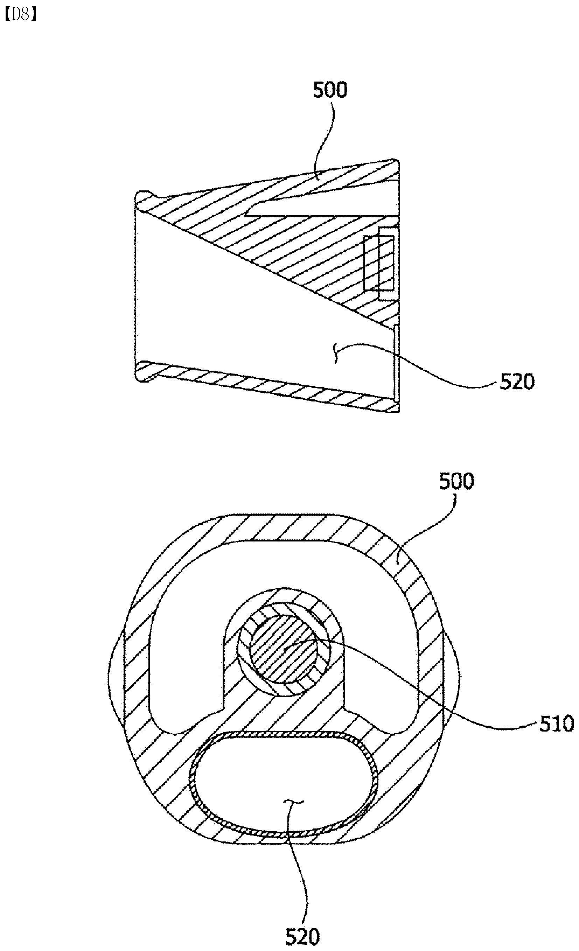

The mouthpiece 500 is a member provided with a respiration hole 520 formed therein and connected to the air flow path 110 so that the user may breathe into the respiration hole 520 in his/her mouth, and a magnetic body 510 is installed at one side of the mouthpiece 500 so as to be detachably coupled to the metal body 410 of the mouthpiece coupling member 400, as shown in FIG. 8. As such, the mouthpiece 500 is detachably coupled to the mouthpiece coupling member, and thereby, the inside of the respiration hole 520 and the filter 520 may be easily cleaned. Here, the magnetic body and the metal body may be interchangeably disposed at the mouthpiece coupling member and the mouthpiece.

The respiration measurement device may further include a respiratory pressure adjustment unit 600 configured to adjust the pressure of respiratory air flow introduced into the respiration measurement device, as needed.

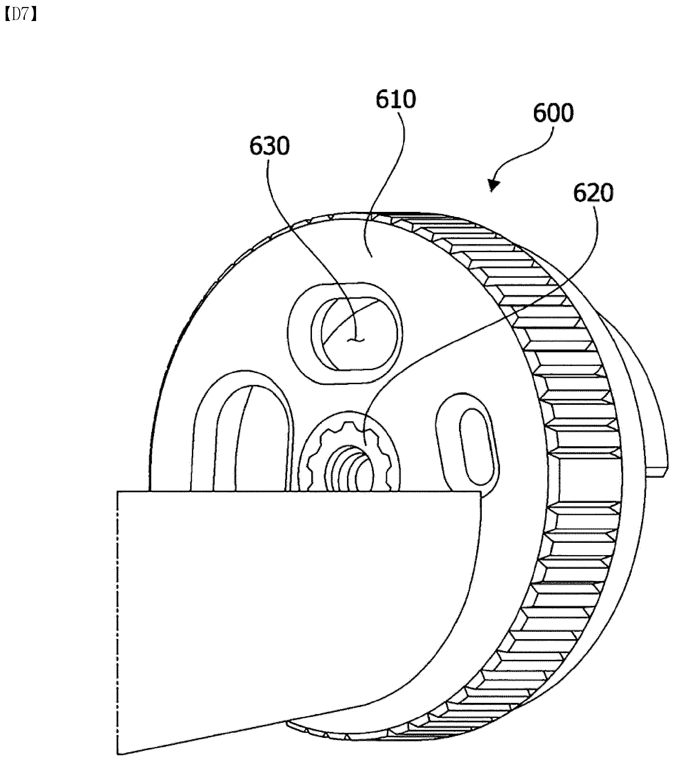

The respiratory pressure adjustment unit 600 includes, as shown in FIG. 7, a rotating member 610, a rotating shaft 620 and a plurality of pressure adjustment holes 630.

The rotating member 610 is a circular plate having a designated thickness, and may be disposed between the inlet side 111 of the air flow path 110 and the mouthpiece coupling member 400. Here, the rotating shaft 620 is provided at the center of the rotating member 610 so that the rotating member 610 can be rotated by the rotating shaft 620.

The pressure adjustment holes 630 are holes formed through the rotating member 610, and are provided in plural so as to be disposed radially about the rotating shaft 620. Here, the cross-sectional areas of the respective pressure adjustment holes 630 are different.

In the respiratory pressure adjustment unit 600 having the above-described configuration, a corresponding one of the different pressure adjustment holes 630 is interlocked with the air flow path 110 depending on rotation of the rotating member 610, and the pressure of the respiratory air flow introduced into the air flow path 110 is adjusted depending on the cross-sectional area of the corresponding pressure adjustment hole 630.

The respiration measurement device having the above-described configuration according to the present invention has a reduced size so that the respiration volume of an examinee can be measured anytime and anywhere as well as at home or in a hospital, and allows the respiration measurement sensor module 310 configured to measure the respiratory air flow and the air flow path 100 configured to allow the respiratory air flow to be moved therethrough to be separately provided and then to be detachably coupled to each other, so that foreign substances produced in the respiration measurement sensor module 310 and the air flow path 110 can be easily washed and removed. Particularly, the first body through which the user's respiratory air flow directly passes is configured to be separable from the second body so as to be very easily washed, and the respiration measurement sensor module of the second body is formed integrally with the sensing unit so that, even when attachment and detachment between the first and second bodies are repeated, a sensing error rarely occurs.

Further, one or more differential pressure generation blocks are formed in the air flow path as a substitute for the conventional orifice pipe, and thereby, the respiration measurement device may increase sensitivity while having a simplified structure.

The respiratory pressure adjustment unit 600 may adjust the pressure of the respiratory air flow introduced into the air flow path 110 depending on the cross-sectional area of a corresponding one of the respective pressure adjustment holes 630, and may thus provide a respiratory pressure suitable for the user's health condition during user's respiratory movement, thereby being capable of improving the respiratory movement effects of individuals.

The respiration measurement device according to the preferred embodiment of the present invention has been described.

Although the above-described embodiment of the present invention has been disclosed for illustrative purposes, those skilled in the art will appreciate that various modifications, additions and substitutions are possible, without departing from the scope of the invention as disclosed in the accompanying claims.

(Description of Reference Numerals and Marks)

100: first body 110: air flow path
120: opening 200: second body
300: respiration measurement unit 310: respiration measurement sensor module
311: sensing flow path 312: sensing unit
320: first differential pressure generation block
330: second differential pressure generation block

340: substrate 350: third differential pressure generation block
360: fourth differential pressure generation block
400: mouthpiece coupling member
410: metal body 420: filter
500: mouthpiece 510: magnetic body
520: respiration hole 600: respiratory pressure adjustment unit
610: rotating member 620: rotating shaft
630: pressure adjustment hole

**Claims**

1. A respiration measurement device comprising:

a first body (100) configured to have an air flow path (110) formed therein to allow air generated by respiration of a user to be introduced thereinto and moved therethrough, a part of the air flow path (110) being opened to form an opening (120);
a second body (200) comprising a respiration measurement unit (300) configured to measure a pressure of the air moved through the air flow path, path(110), formed to be detachably coupled to the first body and installed to close the opening formed through the first body, wherein the respiration measurement unit comprises:

a respiration measurement sensor module (310) formed to correspond to the opening of the air flow path (110) and installed to close the opening of the air flow path (110) by coupling between the first body and the second body; and
a first differential pressure generation block (320) as a member having a designated thickness and height, disposed at the center of the respiration measurement sensor module (310) and configured to extend from the respiration measurement sensor module towards an inner center of the air flow path (110) and the respiration measurement device further comprising :

a second differential pressure generation block (330) as a member having a designated thickness and height, formed to correspond to the first differential pressure generation block(320), disposed on the inner surface of the air flow path(110), configured to extend from an inner surface of the air flow path towards the inner center of the air flow path(110), and disposed opposite the first differential pressure generation block so as to correspond to the first differential pressure generation block; and the respiration measurement unit further comprising
a mouthpiece (500) detachably coupled to an inlet side of the air flow path.

2. The respiration measurement device according to claim 1, wherein the respiration measurement sensor module (310) comprises:

a sensing flow path (311) configured to pass through an inside of the respiration measurement sensor module (310) and provided with an inlet and an outlet formed at both sides of the first differential pressure generation block(320); and
a sensing unit (312) configured to sense a pressure difference between the inlet and the outlet of the sensing flow path(311).

**Patentansprüche**

1. Atmungsmessgerät, umfassend:

einen ersten Korpus (100), der so konfiguriert ist, dass ein Luftströmungsweg (110) darin ausgebildet ist, um durch Atmung eines Anwenders erzeugte Luft einleiten und hindurchbewegen zu können, wobei ein Teil des Luftströmungswegs (110) geöffnet ist, wobei eine Öffnung (120) entsteht;
einen zweiten Korpus (200), der eine Atmungsmesseinheit (300) umfasst, die so konfiguriert ist, dass sie den Druck der durch den Luftströmungsweg (110) bewegten Luft messen kann, der so geformt ist, dass sie lösbar an den ersten Korpus gekoppelt ist, und so montiert ist, dass die durch den ersten Korpus gebildete Öffnung geschlossen wird,

wobei die Atmungsmesseinheit Folgendes umfasst:

ein Atmungsmessungssensormodul (310), das so geformt ist, dass es der Öffnung des Luftströmungswegs (110) entspricht, und so montiert ist, dass es die Öffnung des Luftströmungswegs (110) durch Kopplung zwischen dem ersten Korpus und dem zweiten Korpus verschließt; und
einen ersten Differentialdruckerzeugungsblock (320) als Element mit einer definierten Dicke und Höhe, das sich in der Mitte des Atmungsmessungssensormoduls (310) befindet und so konfiguriert ist, dass es sich ausgehend von dem Atmungsmessungssensormodul zu einem inneren Zentrum des Luftströmungswegs (110) und des Atmungsmessgeräts erstreckt, weiterhin umfassend:

einen zweiten Differentialdruckerzeugungsblock (330) als Element mit einer definierten Dicke und Höhe, das so geformt ist, dass es dem ersten Differentialdruckerzeugungsblock (320) entspricht, und sich auf der Innenfläche des Luftströmungswegs (110) befindet, so konfiguriert ist, dass es sich ausgehend von einer Innenfläche des Luftströmungswegs zu dem inneren Zentrum des Luftströmungswegs (110) erstreckt, und sich dem ersten Differentialdruckerzeugungsblock gegenüber befindet, so dass er dem ersten Differentialdruckerzeugungsblock entspricht;
und
die Atemmesseinheit weiterhin ein Mundstück (500) umfasst, das lösbar an eine Einlassseite des Luftströmungswegs gekoppelt ist.

2. Atemmessgerät gemäß Anspruch 1, wobei das Atmungsmessungssensormodul (310) Folgendes umfasst:

einen Sensorströmungsweg (311), der so konfiguriert ist, dass er durch das Innere des Atmungsmessungssensormoduls (310) verläuft, und mit einem Einlass und einem Auslass versehen ist, die auf beiden Seiten des ersten Differentialdruckerzeugungsblocks (320) ausgebildet sind; und
eine Sensoreinheit (312), die so konfiguriert ist, dass sie eine Druckdifferenz zwischen dem Einlass und dem Auslass des Sensorströmungswegs (311) erfassen kann.

**Revendications**

1. Dispositif de mesure de respiration, comprenant :

un premier corps (100) configuré pour avoir une voie d'écoulement d'air (110) formée dedans pour laisser de l'air produit par respiration d'un utilisateur être introduit dedans et passé à travers, une partie de la voie d'écoulement d'air (110) étant ouverte pour former une ouverture (120),
un deuxième corps (200) comprenant une unité de mesure de respiration (300) configurée pour mesurer une pression de l'air passé à travers la voie d'écoulement d'air (110), formé pour être couplé de manière détachable audit premier corps, et monté pour fermer l'ouverture formée à travers le premier corps,
dans lequel ladite unité de mesure de respiration comprend :

un module capteur de mesure de respiration (310) formé pour correspondre à l'ouverture de la voie d'écoulement d'air (110), et monté pour fermer l'ouverture de la voie d'écoulement d'air (110) par couplage entre le premier corps et le deuxième corps, et
un premier bloc de génération de pression différentielle (320) comme élément ayant une épaisseur et hauteur désignées, disposé au centre dudit module capteur de mesure de respiration (310), et configuré pour s'étendre à partir du module capteur de mesure de respiration vers un centre intérieur de la voie d'écoulement d'air (110) et le dispositif de mesure de respiration, comprenant en outre :

un deuxième bloc de génération de pression différentielle (330) comme élément ayant une épaisseur et hauteur désignées, formé pour correspondre au premier bloc de génération de pression différentielle (320), disposé sur la surface intérieure de la voie d'écoulement d'air (110), configuré pour s'étendre à partir d'une surface intérieure de la voie d'écoulement d'air vers le centre intérieur de la voie d'écoulement d'air (110), et disposé en face du premier bloc de génération de pression différentielle pour correspondre au premier bloc de génération de pression différentielle,
et
ladite unité de mesure de respiration comprenant en outre un embout (500) couplé de manière détachable à un côté d'entrée de la voie d'écoulement d'air.

**2.** Dispositif de mesure de respiration selon la revendication 1, dans lequel un module capteur de mesure de respiration (310) comprend :

une voie d'écoulement du capteur (311) configurée pour passer à travers l'intérieur dudit module capteur de mesure de respiration (310), et munie avec une entrée et une sortie formées au deux côtés dudit premier bloc de génération de pression différentielle (320), et
une unité de détection (312) configurée pour observer une différence de pression entre l'entrée et la sortie de ladite voie d'écoulement du capteur (311).

【D1】

【D2】

【D3】

120

100

【D4】

500 400 600 340 312 311 200

310

110

100

111 330 320

300

【D5】

【D6】

【D7】

【D8】

【D9】

【D10】

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5137026 A